# EUROPÄISCHE PATENTSCHRIFT

(11) **EP 0 927 180 B1**
(45) Veröffentlichungstag und Bekanntmachung des Hinweises auf die Patenterteilung: **08.08.2007**
(21) Anmeldenummer: 97909251.7
(22) Anmeldetag: 11.09.1997
(51) Int. Cl.: C07D 405/04, C07D 413/04, C07F 9/6558, C12Q 1/68, C07H 21/04

(54) **HETEROCYCLISCHE VERBINDUNGEN UND DEREN VERWENDUNG IN DER DETEKTION VON NUCLEINSÄUREN**
HETEROCYCLIC COMPOUNDS AND THEIR USE FOR ISOLATING NUCLEIC ACIDS
COMPOSES HETEROCYCLIQUES ET LEUR UTILISATION POUR LA MISE EN EVIDENCE D'ACIDES NUCLEIQUES

(30) Priorität: 12.09.1996 DE 19637042
(43) Veröffentlichungstag der Anmeldung: 07.07.1999
(73) Patentinhaber: Roche Diagnostics GmbH, 68305 Mannheim (DE)
(72) Erfinder: MÜHLEGGER, Klaus, D-82398 Polling (DE); VON DER ELTZ, Herbert, D-82362 Weilheim (DE)
(86) Internationale Anmeldenummer: PCT/EP1997/004972
(87) Internationale Veröffentlichungsnummer: WO 1998/011104

(56) Entgegenhaltungen:
- EP-A- 0 063 879
- EP-A- 0 359 225
- EP-A- 0 371 262
- EP-A- 0 399 330
- WO-A-93/16094
- GRIERSON J R ET AL: "RADIOSYNTHESES OF LABELED 2PSEUDOTHYMIDINE* ( C-11- AND H-3METHYL) AND ITS BIODISTRIBUTION AND METABOLISM IN NORMAL AND TUMORED MICE" NUCLEAR MEDICINE AND BIOLOGY, Bd. 22, Nr. 5, 1.Juli 1995, Seiten 671-678, XP000512448

## Beschreibung

Die Erfindung betrifft heterocyclische Verbindungen, die zur Markierung, Detektion und Sequenzierung von Nucleinsäuren eingesetzt werden können.

Nucleinsäuren haben als Träger bzw. Überträger der genetischen Information eine zentrale Bedeutung in der belebten Natur. Sie haben deshalb seit ihrer Entdeckung durch F. Miescher das breite Interesse der Naturwissenschaften erregt und zur Aufklärung ihrer Funktion, Struktur und Wirkungsweise geführt.

Ein wesentliches Werkzeug zur Erklärung dieser Zusammenhänge und der Lösung der Probleme war und ist der Nachweis der Nucleinsäuren und zwar sowohl was ihren spezifischen Nachweis betrifft, als auch was ihre Sequenz, also ihre Primärstruktur angeht.

Die spezifische Nachweisbarkeit von Nucleinsäuren beruht auf der Eigenschaft dieser Moleküle, mit anderen Nucleinsäuren durch Ausbildung von Basenpaarungen über Wasserstoffbrücken in Wechselwirkung zu treten, zu "hybridisieren". In geeigneter Weise markierte, d. h. mit Indikatorgruppen versehene Nucleinsäuren ("Sonden") können so zum Nachweis komplementärer Nucleinsäuren ("target") eingesetzt werden.

Die Ermittlung der Primärstruktur ("Sequenz"), also der Abfolge der heterocyclischen Basen einer Nucleinsäure geschieht mittels den Techniken der "Sequenzierung". Diese Kenntnis der Sequenz ist wiederum die Grundvoraussetzung für einen gezielten und spezifischen Einsatz von Nucleinsäuren in molekularbiologischen Fragestellungen und Arbeitstechniken. Auch die Sequenzierung bedient sich letztlich des Prinzips der spezifischen Hybridisierung von Nucleinsäuren untereinander. Dabei werden wie oben erwähnt ebenfalls markierte Nucleinsäure-Fragmente verwendet.

Die geeignete Markierung von Nucleinsäuren ist also eine unverzichtbare Voraussetzung jeglicher Nachweismethode.

Schon frühzeitig wurde dafür vor allem die radioaktive Markierung mit geeigneten Isotopen, wie ³²P oder ³⁵S eingesetzt. Die Nachteile der Verwendung radioaktiver Reagentien liegen jedoch klar auf der Hand: entsprechende Arbeiten bedürfen spezieller räumlicher Einrichtungen und Genehmigungen, sowie einer kontrollierten und aufwendigen Entsorgung des radioaktiven Abfalls. Die Reagentien zur radioaktiven Markierung sind teuer. Eine längere zeitliche Aufbewahrung derart markierter Proben ist wegen der kurzen Halbwertszeit obiger Nuklide nicht möglich.

Es hat daher in den letzten Jahren nicht an Versuchen gefehlt, diese gravierenden Nachteile zu umgehen, d. h. von einer radioaktiven Markierung wegzukommen. Dabei sollte die hohe Sensitivität dieser Markierungsart möglichst beibehalten werden.

Hier sind in der Tat bereits große Fortschritte erzielt worden [siehe z. B. "Nonradioactive Labeling and Detection of Biomolecules" (Kessler, C., Hrsg.) Springer Verlag Berlin, Heidelberg 1992].

Eine unabdingbare Voraussetzung jeglicher Detektion einer Nucleinsäure ist die vorherige Markierung derselben. Wie oben angedeutet, ist es wünschenswert, dies in einer nicht-radioaktiven Art und Weise zu erreichen. Während die radioaktive Markierung der Nuclein-säuren in der Regel durch enzymatisch katalysierten Einbau entsprechender radioaktiver Nucleosidtriphosphate erfolgt, muß eine nicht-radioaktive Markierung über den Einbau einer geeigneten Signal- oder Reportergruppe geschehen.

Als nicht-radioaktive Indikatormoleküle haben sich u. a. hauptsächlich Haptene (wie Biotin oder Digoxigenin), Enzyme (wie alkalische Phosphatase oder Peroxidase) oder Fluoreszenz-farbstoffe (wie Fluorescein oder Rhodamine) bewährt. Diese Signalgruppen können nach verschiedenen Methoden an oder in Nucleinsäuren gebracht werden.

Eine relativ einfache Verfahrensweise z. B. ist die Markierung am 5'-Ende eines mit einer endständigen Aminofunktion versehenen Oligonucleotides mittels aktivierter Indikatormoleküle der oben genannten Art. Sie erlaubt aber nur das Einführen eines oder weniger Indikatormoleküle in ein nur niedermolekulares Oligomer, während eine dichtere Markierung längerkettiger, hochmolekularer Nucleinsäuren mit dem Ziel einer hohen Sensitivität in der Regel über den Einbau von mit Reportergruppen versehenen Nucleosid-triphosphaten mittels Polymerasen im Sinne einer *de novo* -Synthese erfolgen muß.

Solche gängigen Verfahren sind dem Fachmann als "nick translation" [Rigby, P. W. et al. (1977) J. Mol. Biol. 113, 237] und "random primed labeling" [Feinberg, A. P. & Vogelstein, B. (1984) Anal. Biochem. 137, 266] bekannt. Eine weitere Methode ist die sogenannte 3' -tailing - Reaktion mit Hilfe des Enzyms "Terminale Transferase"[z. B. Schmitz, G. et al. (1991) Anal. Biochem. 192, 222].

Die bislang in diesen Verfahren eingesetzten Nucleosid-triphosphate sind nahezu ausschließlich entsprechend modifizierte Derivate der heterocyclischen Basen Adenin, Guanin, Cytosin und Thymin in der Desoxyribonucleotid-, bzw. Adenin, Guanin, Cytosin und Uracil in der Ribonucleotid-Reihe. Solche Derivate sind z. B. von Langer et al. in Proc. Natl. Acad. Sci. USA 78, 6635 (1981), Mühlegger et al. Biol. Chem. Hoppe-Seyler 371, 953 (1990) und in EP 0 063 879 beschrieben. Hier werden die natürlicherweise in DNA und RNA vorkommenden Bausteine in markierter, d. h. mit Signalgruppen versehener Form eingesetzt.

Die wesentlichsten Nachteile dieser N-Nucleoside liegen in der Empfindlichkeit der N-glykosidischen Bindung gegenüber sauren pH-Bedingungen und der Abbaubarkeit durch Nucleasen.

Ferner sind einzelne C-Nukleoside (siehe z. B. Suhadolnik, R.J. in "Nucleoside Antibiotics", Wiley-Interscience, New York 1970) und deren Verwendung im therapeutischen (antiviralen oder cancerostatischen) Bereich seit längerem bekannt.

Außerdem sind fluoreszierende C-Nukleosid-Derivate und deren Einbau in DNA- bzw. RNA-Oligonukleotide beschrieben (WO 93/16094). Die sogenannte Eigenfluoreszenz dieser C-Nukleoside ist jedoch bezüglich der Quantenausbeute um ein Vielfaches geringer als die spezieller Fluorophore wie z. B. Fluorescein oder entsprechende Rhodaminderivate. Ein weiterer Nachteil der selbstfluoreszenten C-Nukleoside liegt in ihren vergleichsweise niedrigen Anregungs- und Emissionswellenlängen. Dies hat zur Folge, daß Detektionssysteme, welche auf solchen Derivaten beruhen, nur eine geringe Nachweisempfindlichkeit besitzen, zum anderen machen sich spektral störende Einflüsse der Meßumgebung (wie biologisches Material, Autofluoreszenz von Gelmatrices etc.) sehr stark bemerkbar.

Die bekannten Nukleoside bzw. Nukleosid-Derivate weisen somit eine Reihe von Nachteilen auf, die sich insbesondere für den Nachweis von Nukleinsäuren negativ auswirken.

Der Erfindung liegt somit die Aufgabe zugrunde mit Signalgruppen modifizierte Nukleosid-Derivate für die Detektion von Nukleinsäuren zur Verfügung zu stellen, die die genannten Nachteile nicht aufweisen, d. h. insbesondere stabiler sowie zugleich enzymatisch prozessierbar und für den Nachweis von Nukleinsäuren bei einer praktikablen Wellenlänge geeignet sind.

Die Aufgabe wird durch heterocyclische Verbindungen der allgemeinen Formel I gelöst: 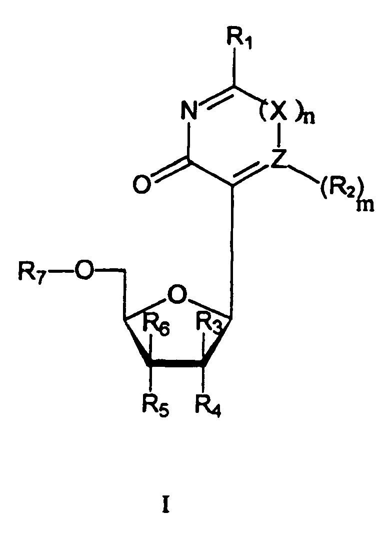 in der
- R₁ und R₂,: die gleich oder verschieden sein können, Wasserstoff, Sauerstoff, Halogen, Hydroxy, Thio- oder substituiertes Thio-, Amino- oder substituiertes Amino-, Carboxy-, Niederalkyl-, Niederalkenyl-, Niederalkinyl-, Aryl-, Niederalkyloxy-, Aryloxy-, Aralkyl-, Aralkyloxy-, oder eine Reportergruppe darstellen,
- R₃ und R₄: jeweils Wasserstoff, Hydroxy, Thio- oder substituiertes Thio-, Amino- oder substituiertes Amino-, Niederalkyloxy-, Niederalkenoxy-, Niederalkinoxy-, eine Schutzgruppe oder eine Reportergruppe darstellen.
- R₅: Wasserstoff, Hydroxy, Thio- oder substituierte Thio-, Amino- oder substituierte Amino-Gruppe, reaktive 3- oder 5-bindige Phosphorgruppe wie z. B. eine Phosphoramidit- oder H-phosphonat-Funktion, einen in geeigneter Weise spaltbaren Ester- oder Amid-Rest oder eine Reportergruppe darstellt,
- R₄ und R₅: zusammen eine weitere Bindung zwischen C-2' und C-3' oder eine Acetalfunktion bilden.
- R₆: Wasserstoff oder eine Hydroxy-, Thio- oder substituierte Thio-, Amino- oder substituierte Amino-Gruppe darstellt,
- R₇: Wasserstoff, eine Mono-, Di- oder Triphosphatgruppe, oder die alpha-, beta- oder gamma- Thiophosphatanalogen dieser Phosphorsäureester oder eine Schutzgruppe darstellt,
sowie mögliche Tautomere und Salze derselben.

X mit Halogen, Hydroxy, Thio- oder substituiertes Thio-, Amino- oder substituiertes Amino-, Carboxy-, Niederalkyl-, Niederalkenyl-, Niederalkinyl-, Aryl-, Niederalkyloxy-, Aryloxy-, Aralkyl-, Aralkyloxy- oder einer Reportergruppe substituiertes Methylen oder Methin oder Sauerstoff und n=0 oder 1 bedeuten,

Z Stickstoff oder Kohlenstoff darstellen, mit der Maßgabe, daß wenn Z Stickstoff bedeutet, m null (0) ist, und wenn X Methylen, substituiertes Methylen oder substituiertes Methin darstellt, Z nicht Kohlenstoff sein kann, und wenn X Sauerstoff bedeutet, Z nicht Stickstoff sein kann, wobei die Verbindung mit einer Reportergruppe modifiziert ist.

Als Reportergruppe kommen jegliche detektierbaren Gruppen, wie insbesondere Haptene, ein Fluorophor, eine Metall-chelatierende Gruppe, ein Lumiphor, ein Protein- oder ein Interkalator in Frage.

Bevorzugt sind solche Verbindungen der allgemeinen Formel I, in denen die Acetalfunktion der Reste R₄ und R₅ mit einer Reportergruppe substituiert ist. Die Reportergruppe kann direkt oder indirekt d.h. über Linkerfunktion gebunden sein.

Des weiteren haben sich solche Verbindungen der allgemeinen Formel I als besonders geeignet erwiesen, in denen R₁ Sauerstoff R₂ Wasserstoff oder eine Reportergruppe, R₃ und R₄ Wasserstoff, R₁ Hydroxy, Wasserstoff, eine reaktive 3- oder 5- bindige Phosphorgruppe, R₆ Wasserstoff und R₇ Wasserstoff, Mono-, Di-, Triphosphat-Gruppen darstellen kann.

Weiterhin bevorzugt sind Verbindungen der allgemeinen Formel I, in denen die Reportergruppe über eine sogenannte Linkergruppe an den heterocyclischen- bzw. Tetrahydro-furan-Ring gebunden ist. Entsprechende Linkergruppen sind dem Fachmann bekannt (siehe z. B. Mühlegger, K. et al. (1990) Biol. Chem. Hoppe-Seyler 371, 953-965 oder Livak, K. J. et al. (1992) Nucl. Acids Res. 20, 4831-4837).

Weiterhin bevorzugt sind Verbindungen der allgemeinen Formel I, in denen R₁ Wasserstoff, Hydroxy, eine Aminogruppe, eine gegebenenfalls substituierte Aminogruppe oder eine Reportergruppe, R₂ eine gegebenenfalls substituierte Aminogruppe oder eine Reportergruppe, R₃ Wasserstoff, R₄ Wasserstoff, Hydroxy, Amino- oder substituiertes Amino-, Niederalkyloxy-, Niederalkenoxy-, Niederalkinoxy-, R₅ Wasserstoff, Hydroxy, Thio, eine gegebenenfalls substituierte Aminogruppe, ein Phosphoramidit oder eine Reportergruppe, R₄ und R₅ zusammen eine Acetalfunktion, R₆ Wasserstoff und R₇ eine Triphosphat-Funktion darstellen.

Bevorzugt sind auch die Verbindungen nach Formel I, in denen X Sauerstoff bedeutet und gleichzeitig Z mit R₂ substituierten Kohlenstoff darstellt, oder Z Stickstoff bedeutet und gleichzeitig X mit Amino- oder substituiertem Amino-, Carboxy-, oder einer Reportergruppe substituiertes Methylen oder Methin darstellen.

Eine weiter bevorzugte Ausführungsform sind Verbindungen nach Formel I, in denen X = 0 ist und Z mit Amino- oder substituiertem Amino-, Carboxy-, oder einer Reportergruppe substituiertes Methin darstellen.

Die Synthese der erfindungsgemäßen Verbindungen kann auf verschiedene Weise erfolgen. Zum Teil kann von natürlich vorkommenden Vorstufen ausgegangen werden, wie beispiels-weise von 3-(3,4-Dihydroxy-5-hydroxymethyl-tetrahydrofuran-2-yl)-pyrrol-2,5-dion oder 3-(3,4-Dihydroxy-5-hydroxymethyl-tetrahydrofuran-2-yl)-oxazin-2,6-dion.

Die Synthese der wichtigen 3-(3-Desoxy-4-hydroxy-5-hydroxymethyl-tetrahydrofuran-2-yl)-Derivate erfolgt aus diesen Vorstufen durch Deoxigenierung, vorzugsweise nach Barton (Barton, D. H. R. & Motherwell, W. B. (1981) Pure Appl. Chem. 53, 15).

Des weiteren kann die chemische Synthese der neuen heterocyclischen Verbindungen wie beispielsweise von K. A. Watanabe in "Chemistry of Nucleosides and Nucleotides" 3, 421-535 (L. B. Townsend, Hrsg.), Plenum Press, New York and London, 1994 eingehend beschrieben, erfolgen.

Weitere Synthesen der genannten Ausgangsverbindungen wurden beispielsweise von Hosmane, R. S. et al. in Bioorg. & Med. Chem. Lett. 3, 2847 (1993) und von Townsend, L. B. et al. in Tetrahedron Lett. 36, 8363 (1995) beschrieben.

Die Verwendung der erfindungsgemäßen Verbindungen zur Markierung von Nukleinsäuren mit diversen, definierten Signalgruppen und damit die Detektion und Sequenzierung von Nukleinsäuren hat sich als besonders vorteilhaft erwiesen.

Die erfindungsgemäßen Substanzen der allgemeinen Formeln I besitzen insbesondere gegenüber den klassischen Nukleosiden und Nukleotiden wie Adenosin, Guanosin, Cytidin, Thymidin, Uridin bzw. deren entsprechenden Phosphorsäureestern eine Reihe von Vorteilen.

Ein Vorteil ist die chemische Stabilität z. B. gegenüber sauren pH-Bedingungen. Ein weiterer wesentlicher Vorteil ist die Stabilität dieser Verbindungen gegenüber dem enzymatischen Abbau durch Endo- und Exonukleasen. Diese Enzyme sind in biologischem Material enthalten und können den Nukleinsäure-Nachweis empfindlich stören. Andererseits ist es bekannt, daß DNA- und RNA-Polymerasen kritisch bezüglich der Akzeptanz mehr oder weniger modifizierter Nukleosid-5'-triphosphate sind, d. h. in der *de novo* Synthese solche Nukleotide als Substrate zu erkennen und einzubauen. Insbesondere das Anknüpfen von Signalgruppen an die Nukleotide beeinflußt erfahrungsgemäß Einbau und Einbaurate.

Daß die erfindungsgemäßen Derivate in sehr effizienter Weise durch geeignete Polymerasen in Nukleinsäuren eingebaut werden, wie z. B. nach den oben geschilderten Methoden der "nick translation" oder des "random primed labelling", ist nicht aus dem Stand der Technik zu entnehmen und somit für den Fachmann als überraschend anzusehen.

Der genannten Verfahren bedient man sich ganz allgemein in der Nukleinsäure-Detektion, z. B. beim quantitativen Nachweis nach Blotting-Techniken auf Membranen oder auch in Mikrotiterplatten.

Bei der Sequenzierung, d. h. dem Nachweis der Sequenz einer Nukleinsäure, wird an der zu sequenzierenden Nukleinsäure unter Zuhilfenahme eines kurzen (Start)Oligonukleotides ("primer"), sowie der Zugabe markierter Nucleosid-triphosphate und einer Polymerase ein komplementärer Gegenstrang neusynthetisiert, anschließend sogenannte Terminationsreaktionen ausgeführt und die dabei erzeugten Nukleinsäurefragmente gelchromatographisch aufgetrennt.

In der *in situ* -Hybridisierung zur Detektion bestimmter Gene oder Genomabschnitte läuft in der Zelle im Prinzip das gleiche ab, nämlich der spezifische Einbau von markierten Nukleotiden.

Die oben erwähnten Primer, d. h. kurzkettige Oligonukleotide, sollen, um eine optimale Funktion zu gewährleisten, sowohl stabile Basenpaarungen mit dem Matrizenstrang eingehen, als auch durch endogene Nukleasen nicht angegriffen werden.

Dies wird von Oligonukleotiden, welche anstatt der klassischen Nukleoside die erfindungsgemäßen Verbindungen als Bausteine enthalten, erfüllt.

Gleiches gilt für längerkettige Polynukleotide und Nukleinsäuren, die solche Bausteine beeinhalten. Auch diese sind Gegenstand der vorliegenden Erfindung.

Entsprechende Oligonukleotide, sowie ihre präparativen Vorstufen in Form sogenannter Phosphoramidite und H-phosphonate sind daher ebenfalls Gegenstand der Erfindung.

Oligonukleotide werden heute in der Regel nach bekannten Methoden in automatisch arbeitenden DNA/RNA-Synthesegeräten im Sinne einer Festphasensynthese hergestellt.

Solche Syntheseverfahren basieren im wesentlichen auf der schrittweisen Umsetzung der o. a. Phosphoramidite oder H-Phosphonate und damit der fortlaufenden Verknüpfung dieser monomeren Bausteine zu Oligomeren (siehe z.B. T. Brown & D. J. S. Brown in "Oligonukleotides and Analogues-A Practical Approach" (1991) (Eckstein, F., Hrsg.), IRL Press at Oxford University Press, Oxford, New York, Tokyo).

### Legende

### Abbildung 1:

I und II bedeuten pBR 328-DNA, markiert durch DIG-dUTP-Einbau (Standard), und III pBR 328-DNA, markiert durch die nach Beispiel 6 hergestellte Verbindung 3-(4-Hydroxy-5-triphosphoryl-tetrahydrofuran-2-yl)-4-(digoxigeninyl-3-O-succinylaminocaproylamino-pentyl)-amino-pyrrol-2,5-dion. Die Auftragung auf das Gel erfolgte in Konzentrationen von 10 bis 0.01 pg.

Die folgenden Beispiele erläutern die Erfindung näher:

### Beispiel 1:

### Beispiel 1.1:

### 3-(4-Hydroxy-5-hydroxymethyl-tetrahydrofuran-2-yl)-pyrrol-2,5-dion

Die Verbindung wurde in einer *de novo* Synthese nach Hosmane, R. S. et al. Bioorg. & Med. Chem. Lett. 3, 2847 (1993) hergestellt.
Sie kann alternativ durch Barton-Deoxigenierung [Barton, D. H. R. & Motherwell, W. B. (1981) Pure Appl. Chem. 53, 15] aus dem durch Fermentation gewonnenen 3,4-Dihydroxy-Derivat (Showdomycin) erhalten werden.

### Beispiel 1.2:

### 3-(4-Hydroxy-5-hydroxymethyl-tetrahydrofuran-2-yl)-4-brom-pyrrol-2,5-dion

213 mg (1 mMol) 3-(4-Hydroxy-5-hydroxymethyl-tetrahydrofuran-2-yl)-pyrrol-2,5-dion nach Beispiel 1.1 gewonnen, werden in 25 ml mit Brom bei RT gesättigtem Wasser gelöst und 3 Stunden bei Zimmertemperatur gerührt. Im DC beobachtet man danach nur noch wenig Ausgangsmaterial. Die Lösung wird im Vakuum vom überschüssigen Brom befreit, auf pH 7 eingestellt und zum Öl eingedampft. Man nimmt in wenig Methanol auf und trennt an einer Kieselgel-Säule mit einem Gemisch aus Chloroform/Methanol 8:2.
Nach Eindampfen der Fraktionen werden 140 mg (48 %) eines schwach gelben Öls erhalten.

Elementaranalyse f. C₉H₁₀NO₅Br (MW 292,2): C_{ber} 36,9; H_{ber}, 3,4; N_{ber} 4,8; Br_{ber} 27,4; C_{gef} 37,35; H_{gef} 3,6; N_{gef} 4,5; Br_{gef} 27,8.

### Beispiel 1.3:

### 3-(4-Hydroxy-5-hydroxymethyl-tetrahydrofuran-2-yl)-4-(1,5-diaminopentyl)-pyrrol-2,5-dion

140 mg (ca. 0,5 mMol) der Bromverbindung aus Bsp. 12 werden in 50 ml Ethanol gelöst, mit 1,75 g (ca 10 mMol) Diaminopentan-dihydrochlorid versetzt und während 5 Stunden zum Rückfluß erhitzt. Danach ist die Umsetzung laut DC (Kieselgel, Chloroform/Methanol 8:2) nahezu quantitativ (im Vergleich zur Bromverbindung tieferlaufender Fleck, mit Ninhydrin positiv). Die Reaktionsmischung wird im Vakuum eingedampft und ohne weitere Reinigung in Beispiel 1.4. eingesetzt.

### Beispiel 1.4:

### 3-(4-Hydroxy-5-hydroxymethyl-tetrahydrofuran-2-yl)-4-(N-trifluoracetamidopentyl)-amino-pyrrol-2,5-dion

Der ölige Rückstand aus Beispiel 1.3 (ca. 2 g) wird in 50 ml wasserfreiem Pyridin gelöst, von Ungelöstem abgesaugt und das Filtrat i.V. zur Trockene eingeengt. Man nimmt in 50 ml absolutem Pyridin auf und versetzt mit 0,75 ml (ca. 5 mMol) Trifluoressigsäureanhydrid.. Nach 5-stündigem Stehen bei RT ist die Acylierung lt. DC vollständig.

Die Reaktionslösung wird danach im Vakuum eingedampft und dreimal mit Methanol coevaporiert. Man nimmt in ca. 20 ml Ethanol auf, filtriert und chromatographiert an Kieselgel mit einem Gemisch Chloroform/ Methanol (9:1). Die vereinigten Fraktionen werden eingedampft, der Rückstand in Dioxan aufgenommen und lyophilisiert. Man erhält 110 mg (53 % d. Th.) der gewünschten Verbindung.

Elementaranalyse f. C₁₆H₂₃N₃O₆F₃ (MW 410,4): C_{ber} 46,8; H_{ber} 5,6; N_{ber} 10,2; F_{ber} 13,9; C_{gef}47,35; H_{gef} 5,9; N_{gef} 10,5; F_{gef} 13,8.

### Beispiel 1.5:

### 3-(4-Hydroxy-5-triphosphoryl-tetrahydrofuran-2-yl)-4-(N-trifluoracetamidopentyl)-amino-pyrrol-2,5-dion

40 mg (0,1 mMol) des geschützten Nucleosides aus Beispiel 1.4 werden nach der Methode von Yoshikawa et al. [Tetrahedron Lett. 50, 5065 (1967)] durch Phosphorylierung mit POCl₃ in das 5'-Monophosphat überführt, daraus wird entsprechend dem Verfahren von Hoard & Ott [J. Am. Chem. Soc. 87, (1965)] nach Aktivierung mit Carbonyldiimidazol und Umsetzung mit Pyrophosphorsäure und anschließender IonenaustauschChromatographie an DEAE-Sephadex das gewünschte Triphosphat in einer Ausbeute von 30 mg (46 %)erhalten.

³¹P-NMR (0,1 M EDTA/D₂O/Eth₃N): -5,2 (d, P-γ); -10,3 (d, P-α); -21,0 (t, P-β).

### Beispiel 1.6:

### 3-(4-Hydroxy-5-triphosphoryl-tetrahydrofuran-2-yl)-4-(N-fluoresceinyl-carboxamidopentyl)-amino-pyrrol-2,5-dion

25 mg (0,038 mMol) der trifluoracetyl-geschützten Verbindung aus Beispiel 1.5 werden in 5 ml konz. Ammoniaklösung 1 h bei RT stehen gelassen und anschließend i. V. eingedampft. Der Rückstand wird in 5 ml 0,1 M Boratpuffer, pH 8,5 aufgenommen und mit einer Lösung von 25 mg (0,05 mmol) 5(6)-Carboxy-fluorescein-N-hydroxysuccinimidester in 5 ml aminfreiem Dimethylformamid versetzt. Man lässt über Nacht bei Raumtemperatur stehen. Das Reaktions-gemisch wird auf eine DEAE-Sephadex-Säule (30 x 1 cm) gegeben und mit einem linearen LiCl-Gradient (je 200 ml H₂O auf 0,4 M LiCl) eluiert. Man erhält nach Vereinigen der entsprechenden Frakionen, Eindampfen, Fällung des Konzentrates in Aceton/Ethanol (2:1) und Trocknung 25 mg (ca 50 %) der Titelsubstanz.

| | |
|---|---|
| Spektrale Daten (0, 1 M Phosphatpuffer, pH 9,0): | Excitationₘₐₓ [nm]: 495; |
| | Emissionₘₐₓ: [nm]: 521 |

Entsprechend wurde durch Umsetzung der Verbindung aus Beispiel 1.5 mit Digoxigenin-3-O-succinyl-aminocapronsäure-N-hydroxy-succinimidester das 3-(4-Hydroxy-5-triphosphoryl-tetrahydrofuran-2-yl)-4-(digoxigeninyl-3-O-succinyl-aminocaproylaminopentyl)-amino-pyrrol-2,5-dion hergestellt.

### Beispiel 2:

### Beispiel 2.1:

### 3-(4-Hydroxy-5-hydroxymethyl-tetrahydrofuran-2-yl)-1,3-oxazin-2,6-dion

Die Verbindung wurde durch Barton-Deoxigenierung [Barton, D. H. R. & Motherwell, W. B. (1981) Pure Appl. Chem. 53, 15] aus dem durch Fermentation gewonnenen 3,4-Dihydroxy-Derivat (Oxazinomycin) erhalten.

### Beispiel 2.2:

### 3-(4-Hydroxy-5-hydroxymethyl-tetrahydrofuran-2-yl)-4-brom-1,3-oxazin-2,6-dion

Das Derivat wurde durch Bromierung der Ausgangsverbindung aus Beispiel 2.1 wie in Beispiel 1.2 beschrieben, gewonnen.

### Beispiel 2.3:

### 3-(4-Hydroxy-5-hydroxymethyl-tetrahydrofuran-2-yl)-4-(1,5-diaminopentyl)-1,3-oxazin-2.6-dion

150 mg (0,5 mMol) der Bromverbindung aus Beispiel 2.2 wurden analog dem Verfahren aus Beispiel 1.3 in die Titelverbindung überfuhrt. Diese wurde ohne weitere Reinigung entsprechend den Verfahren der Beispiele 1.4, 1.5 und 1.6 letztlich zum mit Fluorescein markierten Triphosphat umgesetzt.

### Beispiel 3:

### Beispiel 3.1:

### 3-(4-Hydroxy-5-hydroxymethyl-tetrahydrofuran-2-yl)-2,6-diamino-5-chlor-pyrazin

Das Derivat wurde nach Townsend, L. B. et al. Tetrahedron Lett. 36, 8363 (1995) synthetisiert.

### Beispiel 3.2:

3-(4-Hydroxy-5-hydroxymethyl-tetrahydrofuran-2-yl)-2,6-dihydroxy-5-chlor-pyrazin 264 mg (1 mMol) 3-(4-Hydroxy-5-hydroxymethyl-tetrahydrofuran-2-yl)-2,6-diamino-5-chlor-pyrazin aus Beispiel 3.1 wurden einer Desaminierungsreaktion in einem Gemisch aus 50 ml 80 %iger Essigsäure und 700 mg (10 mMol) NaNO₂ unterzogen. Nach 5-stündigem Stehen bei RT war die Umsetzung laut DC nahezu vollständig. Dem Reaktionsansatz wurden zur Zerstörung überschüssigen Nitrits 2 g Harnstoff zugefügt und weitere 3 Stunden bei RT gerührt. Danach wurde die Lösung auf eine Aktivkohle-Säule gegeben (Carboraffin C, ca. 50 ml Volumen), ausreichend gewaschen und das gewünschte Produkt mit Ethanol/Wasser/ Ammoniak eluiert. Nach Eindampfen resultierten 230 mg (ca. 87 %) eines viskosen Öls, das ohne weitere Reinigung in die nächste Stufe eingesetzt wurde.

### Beispiel 3.3:

### 3-(4-Hydroxy-5-hydroxymethyl-tetrahydrofuran-2-yl)-2,6-dihydroxy-5-(1,8-diamino-3,6-dioxa-octyl)-pyrazin

200 mg (0,75 mMol) des Öls aus Beispiel 11 wurden in 30 ml Ethanol gelöst, mit 555 mg (3,75 mMol) 1,8-Diamino-3,6-dioxa-octan versetzt und 3 Stunden auf ca. 60 °C erwärmt.
Danach wurden Lösungsmittel und Amin im Ölpumpenvakuum entfernt und der Rückstand ohne weitere Reinigung durch Umsetzung mit Trifluoressigsäureanhydrid in Pyridin wie unter Beispiel 1.4 beschrieben weiter umgesetzt.

### Beispiel 3.4:

### 3-(4-Hydroxy-5-triphosphoryl-tetrahydrofuran-2-yl)-2,6-dihydroxy-5-[N-trifluoracetamido-(3,6-dioxa)-octyl]-amino-pyrazin

150 mg des trifluoracetylierten Derivates aus Beispiel 3.3 wurden nach Yohikawa und Hoard & Ott wie unter Beispiel 1.5 beschrieben in die Titelverbindung überführt. Nach lonenaustausch-Chromatographie an DEAE-Sephadex resultierte das gewünschte Triphosphat in einer Ausbeute von 120 mg (40 %).

³¹P-NMR (0,1 M EDTA/D₂O/Eth₃N): -5,1 (d, P-γ); - -10,6 (d, P-α); -20,8 (t, P-β).

### Beispiel 3.5:

3-(4-Hydroxy-5-triphosphoryl-tetrahydrofuran-2-yl)-2,6-dihydroxy-5-[N-tetramethylrhodaminyl-5,6-carboxamido-(3,6-dioxa)-octyl]-amino-pyrazin
20 mg des Triphosphates aus Beispiel 3.4 wurden - nach Abspalten der Trinuoracetyl-Schutzgruppe mit Ammoniaklösung (wie in Beispiel 1.6 beschrieben) - mit 20 mg

Tetramethylrhodamin-5(6)-carbonsäure-N-hydroxy-succinimidester in 0,1 M Na-boratpuffer, pH 8,5/DMF wie unter Beispiel 1.6 beschrieben umgesetzt und aufgereinigt.

Es wurden 12 mg des TMR-markierten Produktes erhalten.

| | |
|---|---|
| Spektrale Daten (0,1 M Na-boratpuffer, pH 8,5): | Excitationₘₐₓ [nm]: 551; |
| | Emissionₘₐₓ: [nm]: 575 |

### Beispiel 4:

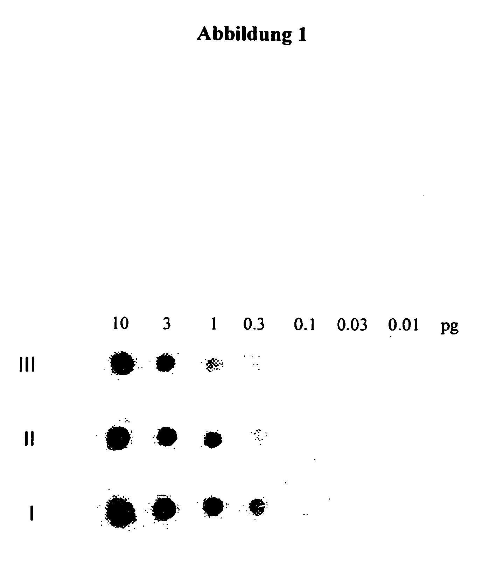

Nichtradioaktive DNA-Markierung und -Nachweis durch Einbau von 3-(4-Hydroxy-5-triphosphoryl-tetrahydrofuran-2-yl)-4-(digoxigeninyl-3-O-succinylaminocaproylamino-pentyl)-amino-pyrrol-2,5-dion
Die DNA-Markierung und der DNA-Nachweis wurden mit dem kommerziell erhältlichen Kit der Firma Boehringer Mannheim (Best. Nr. 1093 657) durchgeführt. Die Arbeitsvorschrift gibt alle wesentlichen Arbeitsschritte wieder.
Zur Markierungsreaktion wurde in dem dNTP-Gemisch des Kits das DIG-dUTP gegen ein 3-(4-Hydroxy-5-triphosphoryl-tetrahydrofuran-2-yl)-4-(digoxigeninyl-3-O-succinylaminocaproylamino-pentyl)-amino-pyrrol-2,5-dion (wie unter Beispiel 1 beschrieben hergestellt) ausgetauscht.
Die immunologische Nachweisreaktion ergab, daß der Einbau der erfindungsgemäßen Verbindung nach Beispiel 1 eine Nachweissensitivität der markierten DNA analog der Verwendung von DIG-dUTP zeigt.
Das Ergebnis, den Nachweis und die erreichte Sensitivität des Systems demonstierend, ist Abbildung 1 zu entnehmen.

## Patentansprüche

1. Verbindungen der allgemeinen Formel 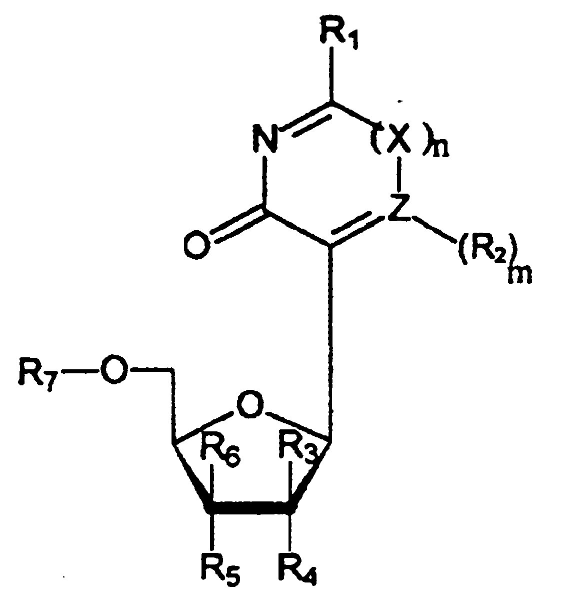 in der
R₁ und R₂, die gleich oder verschieden sein können, Wasserstoff, Sauerstoff, Halogen, Hydroxy, Thio- oder substituiertes Thio-, Amino- oder substituiertes Amino-, Carboxy-, Niederalkyl-, Niederalkenyl-, Niederalkinyl-, Aryl-, Niederalkyloxy-, Aryloxy-, Aralkyl-, Aralkyloxy-, oder eine Reportergruppe darstellen,
R₃ und R₄ jeweils Wasserstoff, Hydroxy, Thio- oder substituiertes Thio-, Amino- oder substituiertes Amino-, Niederalkyloxy-, Niederalkenoxy-, Niederalkinoxy-, eine Schutzgruppe oder eine Reportergruppe darstellen,
R₅ Wasserstoff, Hydroxy, Thio- oder substituierte Thio-, Amino- oder substituierte Amino-Gruppe, eine reaktive 3- oder 5- bindige Phosphorgruppe wie z.B. eine Phosphoramidit- oder H-phosphonat-Funktion, einen in geeigneter Weise spaltbaren Ester- oder Amid-Rest oder eine Reportergruppe darstellt,
R₄ und R₅ zusammen eine weitere Bindung zwischen C-2' und C-3' oder eine Acetalfunktion bilden,
R₆ Wasserstoff oder eine Hydroxy-, Thio- oder substituierte Thio-, Amino- oder substituierte Amino-Gruppe darstellt,
R₇ Wasserstoff, eine Mono-, Di- oder Triphosphatgruppe, oder die alpha-, beta- oder gamma- Thiophosphatanalogen dieser Phosphorsäureester oder eine Schutzgruppe darstellt,
sowie mögliche Tautomere und Salze derselben.
X mit Halogen, Hydroxy, Thio- oder substituiertes Thio-, Amino- oder substituiertes Amino-, Carboxy-, Niederalkyl-, Niederalkenyl-, Niederalkinyl-, Aryl-, Niederalkyloxy-, Aryloxy-, Aralkyl-, Aralkyloxy- oder einer Reportergruppe substituiertes Methylen oder Methin oder Sauerstoff und n=0 oder 1 bedeuten,
Z Stickstoff oder Kohlenstoff darstellen, mit der Maßgabe, dass wenn Z Stickstoff bedeutet, m null (0) ist, und wenn X Methylen, substituiertes Methylen oder substituiertes Methin darstellt, Z nicht Kohlenstoff sein kann, und wenn X Sauerstoff bedeutet, Z nicht Stickstoff sein kann,
wobei die Verbindung mit einer Reportergruppe modifiziert ist.

2. Verbindungen nach Anspruch 1, in denen R₁ Sauerstoff, R₂ Wasserstoff oder eine Reportergruppe, R₃ und R₄ Wasserstoff; R₅ Hydroxy, Wasserstoff; eine reaktive 3- oder 5- bindige Phosphorgruppe, R₆ Wasserstoff und R₇ Wasserstoff; Mono-, Di-, Triphosphat-Gruppen darstellen kann.

3. Verbindungen nach Anspruch 1, in denen R₁ Wasserstoff, Hydroxy, eine Aminogruppe, eine gegebenenfalls substituierte Aminogruppe oder eine Reportergruppe, R₂ eine gegebenenfalls substituierte Aminogruppe oder eine Reportergruppe, R₃ Wasserstoff; R₄ Wasserstoff, Hydroxy, Amino- oder substituiertes Amino-, Niederalkyloxy-, Niederalkenoxy-, Niederalkinoxy-, R₅ Wasserstoff, Hydroxy, Thio, eine gegebenenfalls substituierte Aminogruppe, ein Phosphoramidit oder eine Reportergruppe, R₄ und R₅ zusammen eine Acetalfunktion, R₆ Wasserstoff und R₇ eine Triphosphat-Funktion darstellen.

4. Verbindungen nach Anspruch 1, in denen X Sauerstoff bedeutet und gleichzeitig Z mit R₂ substituierten Kohlenstoff darstellt, oder Z Stickstoff bedeutet und gleichzeitig X mit Amino- oder substituiertem Amino-, Carboxy-, oder einer Reportergruppe substituiertes Methylen oder Methin darstellen.

5. Verbindungen nach Anspruch 1, in denen X = 0 ist und Z mit Amino- oder substituiertem Amino-, Carboxy-, oder einer Reportergruppe substituiertes Methin darstellen.

6. Verbindungen nach Anspruch 1, in denen die Acetalfunktion der Reste R₄ und R₅ mit einer Reportergruppe substituiert ist.

7. Verbindungen nach einem der Ansprüche 1 bis 6, in denen die Reportergruppe ein Hapten, ein Fluorophor, eine Metall-chelatierende Gruppe, ein Luminophor, ein Protein oder ein Interkalator bedeuten.

8. Verbindungen nach einem der Ansprüche 1 bis 7, in denen die Reportergruppe über eine Linkergruppe verknüpft ist.

9. Verwendung der Verbindungen nach einem der Ansprüche 1 bis 8 als Substrate für DNA- und RNA-Polymerasen.

10. Verwendung der Verbindungen nach den Ansprüchen 1 bis 8 zur Markierung von Nukleinsäuren.

11. Verwendung der Verbindungen nach den Ansprüchen 1 bis 8 zum Nachweis von Nukleinsäuren.

12. Verwendung der Verbindungen nach einem der Ansprüche 1 bis 8 in der DNA Sequenzierung.

13. Verwendung der Verbindungen nach einem der Ansprüche 1 bis 8 in der in situ Hybridisierung.

14. Verwendung der Verbindungen nach Anspruch 1 bis 8, in denen R₇ ein Phosphoramidit oder ein H-Phosphonat darstellt, zur chemischen Synthese von Oligonuldeotiden.

15. Oligonukleotide, die eine oder mehrere der in den Ansprüchen 1 bis 8 genannten Verbindungen enthalten.

16. Nukleinsäuren, die eine oder mehrere der in den Ansprüchen 1 bis 8 genannten Verbindungen enthalten.

## Claims

1. Compounds of the general formula 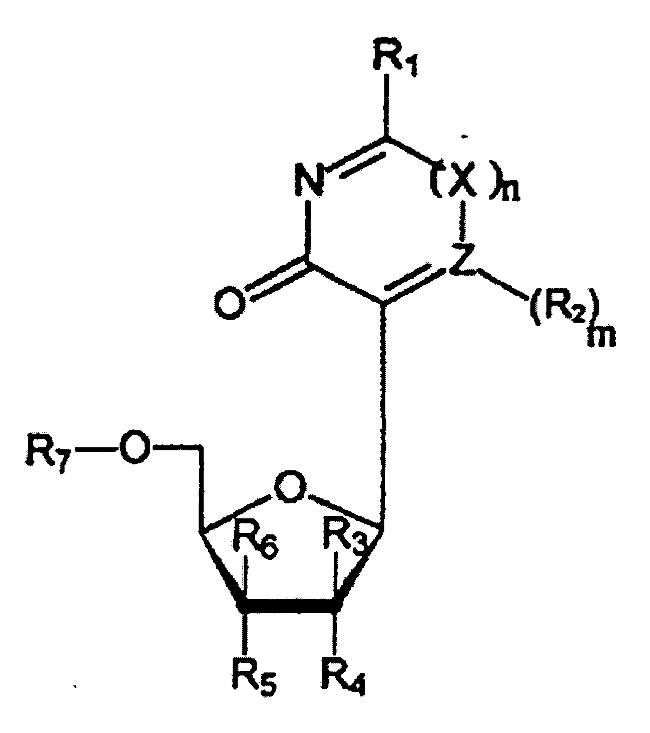 in which
R₁ and R₂ can be the same or different and represent hydrogen, oxygen, halogen, hydroxy, thio or substituted thio, amino or substituted amino, carboxy, lower alkyl, lower alkenyl, lower alkinyl, aryl, lower alkyloxy, aryloxy, aralkyl, aralkyloxy or a reporter group,
R₃ and R₄ each represent hydrogen, hydroxy, thio or substituted thio, amino or substituted amino, lower alkyloxy, lower alkenoxy, lower alkinoxy, a protecting group or a reporter group,
R₅ represents hydrogen, hydroxy, thio or substituted thio, amino or substituted amino group, a reactive trivalent or pentavalent phosphorus group such as e.g. a phosphoramidite or H-phosphonate group, an ester or amide residue that can be cleaved in a suitable manner or a reporter group,
R₄ and R₅ together form a further bond between C-2' and C-3' or an acetal group,
R₆ represents hydrogen or a hydroxy, thio or substituted thio, amino or substituted amino group,
R₇ represents hydrogen, a monophosphate, diphosphate or triphosphate group or the alpha, beta or gamma thiophosphate analogue of this phosphoric acid ester or a protective group
as well as possible tautomers and salts thereof,
X denotes methylene or methine substituted with halogen, hydroxy, thio or substituted thio, amino or substituted amino, carboxy, lower alkyl, lower alkenyl, lower alkinyl, aryl, lower alkyloxy, aryloxy, aralkyl, aralkyloxy or a reporter group, or oxygen and n=0 or 1,
Z represents nitrogen or carbon provided that if Z denotes nitrogen, m is zero (0) and if X represents methylene, substituted methylene or substituted methine, Z cannot be carbon and if X denotes oxygen, Z cannot be nitrogen, wherein the compound is modified with a reporter group.

2. Compounds according to claim 1, in which R₁ can represent oxygen, R₂ can represent hydrogen or a reporter group, R₃ and R₄ can represent hydrogen, R₅ can represent hydroxy, hydrogen, a reactive trivalent or pentavalent phosphorus group, R₆ can represent hydrogen and R₇ can represent hydrogen, mono-, di-, or triphosphate groups.

3. Compounds according to claim 1, in which R₁ represents hydrogen, hydroxy, an amino group, an optionally substituted amino group or a reporter group, R₂ represents an optionally substituted amino group or a reporter group, R₃ represents hydrogen, R₄ represents hydrogen, hydroxy, amino or substituted amino, lower alkyloxy, lower alkenoxy, lower alkinoxy, R₅ represents hydrogen, hydroxy, thio, an optionally substituted amino group, a phosphoramidite or a reporter group, R₄ and R₅ together represent an acetal group, R₆ represents hydrogen and R₇ represents a triphosphate group.

4. Compounds according to claim 1 in which X denotes oxygen and at the same time Z represents carbon substituted with R₂, or Z denotes nitrogen and at the same time X represents methylene or methine substituted with amino or substituted amino, with carboxy or with a reporter group.

5. Compounds according to claim 1 in which X = 0 and Z represents methine substituted with amino or substituted amino, with carboxy or with a reporter group.

6. Compounds according to claim 1, in which the acetal group of the residues R₄ and R₅ is substituted with a reporter group.

7. Compounds according to one of the claims 1 to 6, in which the reporter group denotes a hapten, a fluorophore, a metal-chelating group, a luminophore, a protein or an intercalator.

8. Compounds according to one of the claims 1 to 7, in which the reporter group is linked via a linker group.

9. Use of compounds according to one of the claims 1 to 8 as substrates for DNA and RNA polymerases.

10. Use of compounds according to claims 1 to 8 for labelling nucleic acids.

11. Use of compounds according to claims 1 to 8 for the detection of nucleic acids.

12. Use of compounds according to one of the claims 1 to 8 in DNA sequencing.

13. Use of compounds according to one of the claims 1 to 8 in *in situ* hybridization.

14. Use of compounds according to claims 1 to 8, in which R₇ represents a phosphoramidite or a H-phosphonate for the chemical synthesis of oligonucleotides.

15. Oligonucleotides which contain one or more of the compounds mentioned in claims 1 to 8.

16. Nucleic acids which contain one or more of the compounds mentioned in claims 1 to 8.

## Revendications

1. Composés répondant à la formule générale 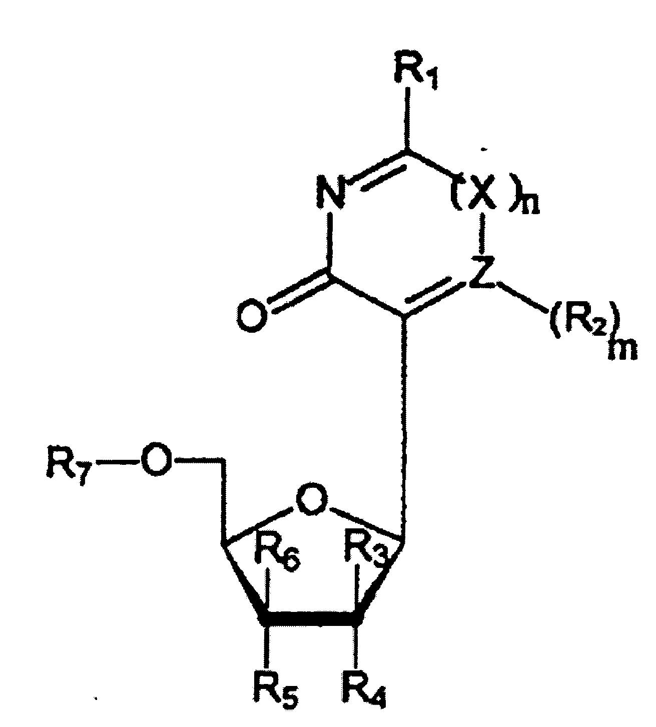 dans laquelle
R₁ et R₂, qui peuvent être identiques ou différents, représentent un atome d'hydrogène, un atome d'oxygène, un atome d'halogène, un groupe hydroxyle, un groupe thio ou un groupe thio substitué, un groupe amino ou un groupe amino substitué, un groupe carboxyle, un groupe alkyle inférieur, un groupe alcényle inférieur, un groupe alcynyle inférieur, un groupe aryle, un groupe alkyle inférieur-oxy, un groupe aryloxy, un groupe aralkyle, un groupe aralkyloxy ou un groupe rapporteur,
R₃ et R₄ représentent respectivement un atome d'hydrogène, un groupe hydroxyle, un groupe thio ou un groupe thio substitué, un groupe amino ou un groupe amino substitué, un groupe alkyle inférieur-oxy, un groupe alcène inférieur-oxy, un groupe alcyne inférieur-oxy, un groupe de protection ou un groupe rapporteur,
R₅ représente un atome d'hydrogène, un groupe hydroxyle, un groupe thio ou un groupe thio substitué, un groupe amino ou un groupe amino substitué, un groupe phosphore réactif à 3 liaisons ou à 5 liaisons comme par exemple une fonction phosphoramidite ou une fonction H-phosphonate, un radical ester ou un radical amide qui peut être scindé de manière appropriée, ou encore un groupe rapporteur,
R₄ et R₅ forment ensemble une liaison supplémentaire entre C-2' et C-3' ou une fonction acétal,
R₆ représente un atome d'hydrogène ou un groupe hydroxyle, un groupe thio ou un groupe thio substitué, un groupe amino ou un groupe amino substitué,
R₇ représente un atome d'hydrogène, un groupe monophosphate, diphosphate ou triphosphate, ou bien les analogues alpha-, bêta- ou gamma-thiophosphates de ces esters d'acide phosphorique ou bien un groupe de protection,
ainsi que leurs sels et leurs tautomères éventuels,
X représente un groupe méthylène ou un groupe méthine substitué avec un atome d'halogène, un groupe hydroxyle, un groupe thio ou un groupe thio substitué, un groupe amino ou un groupe amino substitué, un groupe carboxyle, un groupe alkyle inférieur, un groupe alcényle inférieur, un groupe alcynyle inférieur, un groupe aryle, un groupe alkyle inférieur-oxy, un groupe aryloxy, un groupe aralkyle, un groupe aralkyloxy ou un groupe rapporteur, ou représente un atome d'oxygène, et n = 0 ou 1,
Z représente un atome d'azote ou un atome de carbone, avec cette mesure que, lorsque Z représente un atome d'azote, m est égal à zéro (0), et lorsque X représente un groupe méthylène, un groupe méthylène substitué ou un groupe méthine substitué, Z ne peut pas représenter un atome de carbone, et lorsque X représente un atome d'oxygène, Z ne peut pas représenter un atome d'azote,
le composé étant modifié avec un groupe rapporteur.

2. Composés selon la revendication 1, dans lesquels R₁ peut représenter un atome d'oxygène, R₂ peut représenter un atome d'hydrogène ou un groupe rapporteur, R₃ et R₄ peuvent représenter un atome d'hydrogène, R₅ peut représenter un groupe hydroxyle, un atome d'hydrogène, un groupe phosphore réactif à 3 ou à 5 liaisons, R₆ peut représenter un atome d'hydrogène et R₇ peut représenter un atome d'hydrogène, un groupe monophosphate, diphosphate, triphosphate.

3. Composés selon la revendication 1, dans lesquels R₁ représente un atome d'hydrogène, un groupe hydroxyle, un groupe amino, un groupe amino le cas échéant substitué ou un groupe rapporteur, R₂ représente un groupe amino le cas échéant substitué ou un groupe rapporteur, R₃ représente un atome d'hydrogène, R₄ représente un atome d'hydrogène, un groupe hydroxyle, un groupe amino ou un groupe amino substitué, un groupe alkyle inférieur-oxy, un groupe alcène inférieur-oxy, un groupe alcyne inférieur-oxy, R₅ représente un atome d'hydrogène, un groupe hydroxyle, un groupe thio, un groupe amino le cas échéant substitué, un groupe phosphoramidite ou un groupe rapporteur, R₄ et R₅ représentent ensemble une fonction acétal, R₆ représente un atome d'hydrogène et R₇ représente un groupe triphosphate.

4. Composés selon la revendication 1, dans lesquels X représente un atome d'oxygène et, en même temps, Z représente un atome de carbone substitué avec R₂, ou bien Z représente un atome d'azote et, en même temps, X représente un groupe méthylène ou un groupe méthine substitué avec un groupe amino ou un groupe amino substitué, un groupe carboxyle ou un groupe rapporteur.

5. Composés selon la revendication 1, dans lesquels X représente un atome d'oxygène et Z représente un groupe méthine substitué avec un groupe amino ou un groupe amino substitué, un groupe carboxyle ou un groupe rapporteur.

6. Composés selon la revendication 1, dans lesquels la fonction acétal des radicaux R₄ et R₅ est substituée avec un groupe rapporteur.

7. Composés selon l'une quelconque des revendications 1 à 6, dans lesquels le groupe rapporteur représente un haptène, un fluorophore, un groupe chélatant les métaux, un luminophore, une protéine ou un intercalant.

8. Composés selon l'une quelconque des revendications 1 à 7, dans lesquels le groupe rapporteur est relié via un groupe lieur.

9. Utilisation des composés selon l'une quelconque des revendications 1 à 8, comme substrats pour des ADN- et ARN-polymérases.

10. Utilisation des composés selon l'une quelconque des revendications 1 à 8, pour le marquage d'acides nucléiques.

11. Utilisation des composés selon l'une quelconque des revendications 1 à 8, pour le décèlement d'acides nucléiques.

12. Utilisation des composés selon l'une quelconque des revendications 1 à 8, dans le séquençage d'ADN.

13. Utilisation des composés selon l'une quelconque des revendications 1 à 8, dans l'hybridation in situ.

14. Utilisation des composés selon les revendications 1 à 8, dans laquelle R₇ représente un groupe phosphoramidite ou un groupe H-phosphonate, pour la synthèse chimique d'oligonucléotides.

15. Oligonucléotides qui contiennent un ou plusieurs des composés mentionnés dans les revendications 1 à 8.

16. Acides nucléiques qui contiennent un ou plusieurs des composés mentionnés dans les revendications 1 à 8.
